(19) **Europäisches Patentamt European Patent Office Office européen des brevets**

(11) **EP 3 882 360 B1**

(12) **EUROPEAN PATENT SPECIFICATION**

(45) Date of publication and mention of the grant of the patent:
**06.12.2023 Bulletin 2023/49**

(21) Application number: **20192869.4**

(22) Date of filing: **26.08.2020**

(51) International Patent Classification (IPC):
**C12Q 1/6827** (2018.01)    **G16B 20/00** (2019.01)

(52) Cooperative Patent Classification (CPC):
(C-Sets available)
**C12Q 1/6827; G16B 20/20; G16B 20/40**    (Cont.)

(54) **GENOMIC SELECTION METHOD OF HUAXI CATTLE**

VERFAHREN ZUR GENOMISCHEN SELEKTION VON HUAXI-RINDERN

PROCÉDÉ DE SÉLECTION GÉNOMIQUE DE BOVINS HUAXI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2020 CN 202010192316**

(43) Date of publication of application:
**22.09.2021 Bulletin 2021/38**

(73) Proprietor: **Institute of Animal Sciences of Chinese Academy of Agricultural Sciences Beijing 100193 (CN)**

(72) Inventors:
 • **LI, Junya Beijing, 100193 (CN)**
 • **ZHU, Bo Beijing, 100193 (CN)**
 • **GAO, Huijiang Beijing, 100193 (CN)**
 • **GAO, Xue Beijing, 100193 (CN)**
 • **ZHANG, Lupei Beijing, 100193 (CN)**
 • **XU, Lingyang Beijing, 100193 (CN)**

 • **CHEN, Yan Beijing, 100193 (CN)**
 • **CAI, Wentao Beijing, 100193 (CN)**

(74) Representative: **Acapo AS Edvard Griegs vei 1 5059 Bergen (NO)**

(56) References cited:
**WO-A1-2016/100061**

 • **MA PEIPEI ET AL: "Use of a Bayesian model including QTL markers increases prediction reliability when test animals are distant from the reference population", JOURNAL OF DAIRY SCIENCE, AMERICAN DAIRY SCIENCE ASSOCIATION, US, vol. 102, no. 8, 1 August 2019 (2019-08-01), pages 7237-7247, XP085732128, ISSN: 0022-0302, DOI: 10.3168/JDS.2018-15815 [retrieved on 2019-05-31]**

(52) Cooperative Patent Classification (CPC): (Cont.)

C-Sets
**C12Q 1/6827, C12Q 2537/165**

**Description**

**TECHNICAL FIELD**

The application relates to the technical field of beef cattle breeding, especially relating to a genomic selection method of Huaxi cattle.

**BACKGROUND ART**

The primary factors that limit the development of beef cattle industry in China are the issues of breed and provenance. The existing beef cattle breeding population in China has inadequate capacities in breeding stock and supply, thus the breeding bulls are mainly imported. Among the overall beef cattle amount of livestock, 27.88 million were Huaxi cattle and its hybrid, accounting for 65%. At present, there are 38 breeding bull stations in China for beef bulls, in which 1,900 bulls for semen collection are in stock, and 1,100 are Huaxi cattle. The breeding group can only provide about 100 qualified bulls annually, and about 250 bulls need to be imported annually to update the group, with an import rate up to 70%. Therefore, researches are in process to target the relevant aspects. Document by Ma, Lund, Amand and Su, 2019 discloses a genetic method for predicting economic traits in cattle, based on the use of Bayesian model.
Also document WO2016100061 discloses such a method.

**SUMMARY OF THE INVENTION**

The purpose of the application is to provide a genomic selection method of Huaxi cattle, to solve the problem raised in the background art.
To achieve the above purpose, the embodiments of the application provide the following technical scheme:
A genomic selection method of Huaxi cattle, the specific steps are as follows:

> S1. constructing a reference population and determining 87 economic traits;
> S2. collecting and preserving blood for each individual in the reference population, extracting DNA and genotyping by using biochip, quality control of the data after genotyping;
> S3. proceeding genotype imputation from high density beadchip data to the resequencing data, and then identifying QTLs (quantitative trait loci)for five important economic traits;
> S4. integrating the identified QTLs with the high density beadchip genotyping data to obtain the standard data, calculating the effects of all SNP markers of each trait;
> S5. collecting blood and extracting DNA from a candidate population, genotyping using biochip, proceeding imputation to the standard data after quality control, multiplying the genotype vector of the candidate population by the vector of SNP effects where the effects of all SNP markers and QTLs were estimated by the reference population, thus obtaining the genomic estimated breeding value (GEBV) of the candidate population and calculating the genomic China beef index (GCBI) of the individual according to the genomic estimated breeding value.

As a further scheme of the embodiments in the application: the quality control in S2 is eliminating the unqualified individuals and SNP, biochip is Illumina BovineHD beadchip.
As a further scheme of the embodiments in the application: the economic traits in S1 comprise 6 indexes: growth and development, fattening, slaughter, carcass, meat quality and reproduction, 87 traits in total.
As a further scheme of the embodiments in the application: the QTLs in S3 comprise significant SNPs related to weaning weight, average daily gain during fattening, carcass weight, dressing percentage and calving difficulty.
As a further scheme of the embodiments in the application: the Optimized Bayesian algorithm is used to calculate the effect value of all SNP markers of each trait.
As a further scheme of the embodiments in the application: it is envisaged in the optimized Bayesian algorithm that the probability of SNPs with effect is $(1-\pi)$, the probability of SNPs without effect is $\pi$, and the variance of SNP effects follows the inversed chi-square distribution, its model is as follows:

$$y = Xb + \mathbb{W}v + \sum_{i=1}^{n} Z_i g_i + e$$
,

wherein y is the phenotypic observation vector and b is the fixed effect vector; W is the genotype association matrix of the identified trait QTLs, and v is the locus effect of the identified trait QTLs, which is a fixed effect vector. $g_i$ is the $i^{th}$ marked effect of the 770K beadchip, and the variance is $\sigma^2_{g_i}$ ; $n$ is the total number of markers; $e$ is the random residual vector with a variance of $\sigma^2_e I$ , and $\sigma^2_e$ is the residual variance.

As a further scheme of the embodiments in the application: the equation of individual genomic China beef index (GCBI) in S5 is as follows:

$$GCBI = 100 + (-5 \times \frac{Gebv_{CE}}{1.30} + 35 \times \frac{Gebv_{WWT}}{17.7} + 20 \times \frac{Gebv_{DG\_F}}{0.11} + 25 \times \frac{Gebv_{CW}}{16.4} + 15 \times \frac{Gebv_{DP}}{0.13})$$

where $Gebv_{CE}$ is the genomic estimated breeding value of calving difficulty, $Gebv_{WWT}$ is the genomic estimated breeding value of weaning weight, $Gebv_{DG\_F}$ is the genomic estimated breeding value of average daily gain during fattening, $Gebv_{CW}$ is the genomic estimated breeding value of carcass weight, $Gebv_{DP}$ is the genomic estimated breeding value of dressing percentage.

Compared with the prior art, the embodiments of the invention have the beneficial effects that:

The purpose of the application is to establish a high-efficiency selection method of high-quality Huaxi cattle based on genomic selection technology, comprehensively improve the selection efficiency of beef cattle in China, greatly save the breeding cost, provide a molecular method for selecting cattle for breeding and the cultivation of high-quality Huaxi cattle, and facilitate a rapid development of beef cattle breeding industry.

## BRIEF DESCRIPTION OF THE DRAWINGS

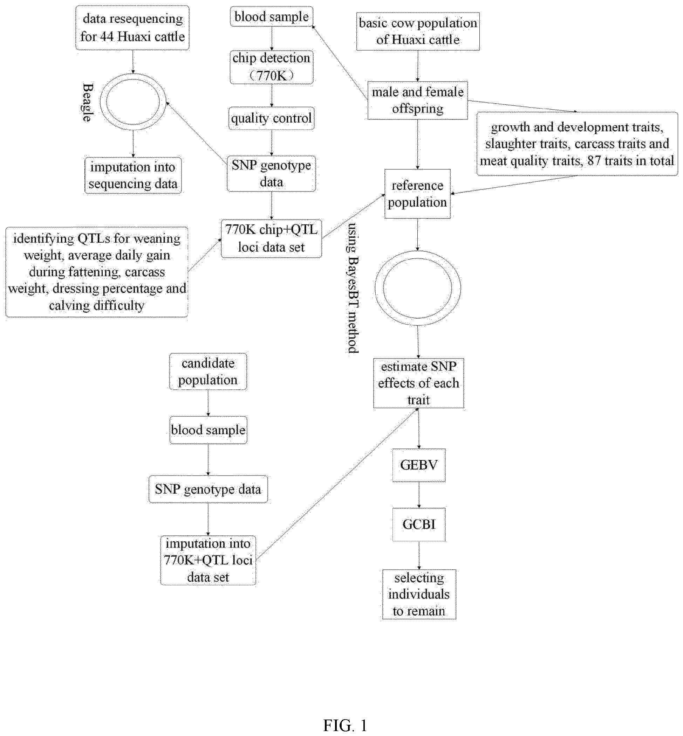

FIG. 1 is a flow chart of genomic selection method of Huaxi cattle.
FIG. 2 is a flow chart showing the improved selection efficiency by genomic selection method of Huaxi cattle.
FIG. 3 is a comparison diagram of SNPs on each autosome before and after data quality control with 770K chip in genomic selection method of Huaxi cattle.
FIG. 4 is a graph showing detection rate of 770K chip markers in genomic selection method of Huaxi cattle.
FIG. 5 is a graph showing the distribution of the minor allele frequency of SNP markers on 770K chip.
FIG. 6 is a probability density diagram of the marker interval on 770K chip in genomic selection method of Huaxi cattle.
FIG. 7 is a graph showing the linkage disequilibrium of the reference population in the genomic selection method in the Huaxi cattle.

## DETAILED DESCRIPTION OF THE EMBODIMENTS

The technical scheme of the application will be described clearly and completely in combination with the embodiments in the application.

## EXAMPLE 1

A genomic selection method of Huaxi cattle, the specific steps were as follows:

S 1. a reference population was constructed and the economic traits were determined: a team started to build 2,320 basic cows of Huaxi cattle in Wulagai Management District of Xilingol League, Inner Mongolia in 2008. With the expanding of the population each year, by 2019, the number of basic cows of Huaxi cattle had exceeded 4,000. The progeny of the basic cow group was used to construct the reference population of Huaxi cattle, and the phenotypic data measured included 6 categories of growth and development, fattening, slaughter, carcass, meat quality and reproduction, and 87 important economic traits in total.

S2. the blood was collected and preserved from each individual in the reference population, DNA was extracted, genotyping was carried out by using Illumina BovineHD (770K) beadchip, and genotyping data was quality controlled by using plink 1.0 software.

S3. the 770K chip data of the reference population were imputed to the resequencing data (16165263SNPs) by using Beagle software with the resequencing data from 44 cows to identify the candidate QTLs for weaning weight, average daily gain during fattening, carcass weight, dressing percentage and calving difficulty.

S4. through SNP genotyping of individuals in the reference population, the screened QTL(quantitative trait locus) loci were integrated with the 770K beadchip loci after quality control, and the 770K chip data were filled into the 770K+QTL locus integrated data by Beagle software. Combined with the phenotypic data, the optimal Bayesian algorithm (BayesBT) based on QTL loci of weaning weight, average daily gain during fattening period, carcass weight, dressing percentage and calving difficulty was used to estimate the effect value of all SNP markers of each trait. It was envisaged in BayesBT that the probability of SNPs with effect is $(1-\pi)$, the probability of SNPs without effect is $\pi$, and the variance of effect obeyed inverse chi-square distribution. The estimation model is as follows:

$$y = Xb + \mathrm{W}v + \sum_{i=1}^{n} Z_i g_i + e,$$

wherein $y$ is a phenotypic observation vector and $b$ is a fixed effect vector; W is the genotype association matrix of the identified trait QTL, and $v$ is the locus effect value of the identified trait QTL, which is a fixed effect vector. $g_i$ is the $i^{th}$ marked effect value of the 770K beadchip, and the variance is $\sigma^2_{g_i}$ ; $n$ is the total number of marks; $e$ is the random residual vector with a variance of $\sigma^2_e I$ , and $\sigma^2_e$ is the residual variance.

S5. the blood was collected from the candidate cattle, DNA was extracted, and genotyped via Illumina Bovine-HD(770K) beadchip. The original chip data was imported into Genome Studio software (reference genome UMD3.1) for quality control to obtain high-quality SNP genotyping data, and the candidate individual genotypes were filled to 770K+QTL locus integration data with Beagle software. The genomic estimated breeding value (GEBV) of the candidate population could be obtained by multiplying the genotype vector of the candidate individual by the locus effect value via utilizing the locus effect value of SNP markers and QTL estimated by the reference population(

$$\mathrm{GEBV_i} = \sum_{i=1}^{n} Z_i g_i + \mathrm{W}v$$

), and the comprehensive selection index (obtained via GCBI) of the individual was calculated according to the genomic estimated breeding value(GEBV) of each trait.

According to the actual situation of domestic breeding data of beef cattle, five important economic traits, namely weaning weight, average daily gain during fattening, carcass weight, dressing percentage and calving difficulty, were selected for genomic evaluation. After standardization, the genomic estimated breeding values of each trait were weighted according to the economic weighting ratio, and the genomic China beef index (GCBI) was obtained. The basic statistics of each trait were shown in Table 1, and the evaluation of accuracy of the genomic breeding value estimation was obtained by 5-fold cross validation with BayesBT calculation software V1.0 for the five quantitative traits.

Table 1. Basic statistics and accuracy of genomic breeding value estimation for each traits

| traits | Number | AVG | SD | accuracy of genomic breeding value estimation |
|---|---|---|---|---|
| weaning weight | 1191 | 163.25 | 57.32 | 0.518 |
| average daily gain during fattening | 1312 | 0.97 | 0.22 | 0.539 |
| carcass weight | 1325 | 276.33 | 48.53 | 0.555 |
| dressing percentage | 1321 | 0.54 | 0.03 | 0.433 |
| calving difficulty | 1081 | 1.33 | 0.49 | 0.487 |

the equation of genomic China beef index (GCBI) is as follows:

$$GCBI = 100 + \left(-5 \times \frac{Gebv_{CE}}{1.30} + 35 \times \frac{Gebv_{WWT}}{17.7} + 20 \times \frac{Gebv_{DG\_F}}{0.11} + 25 \times \frac{Gebv_{CW}}{16.4} + 15 \times \frac{Gebv_{DP}}{0.13}\right),$$

wherein the $Gebv_{CE}$ is the genomic estimated breeding value of calving difficulty, $Gebv_{WWT}$ is the genomic estimated breeding value of weaning weight, $Gebv_{DG\_F}$ is the genomic estimated breeding value of average daily gain during fattening, $Gebv_{CW}$ is the genomic estimated breeding value of carcass weight, $Gebv_{DP}$ is the genomic estimated breeding value of dressing percentage. According to the GCBI of each individual in the candidate population, the candidate individuals were selected for breeding according to the value of this parameter (individuals with GCBI> 120 were selected).

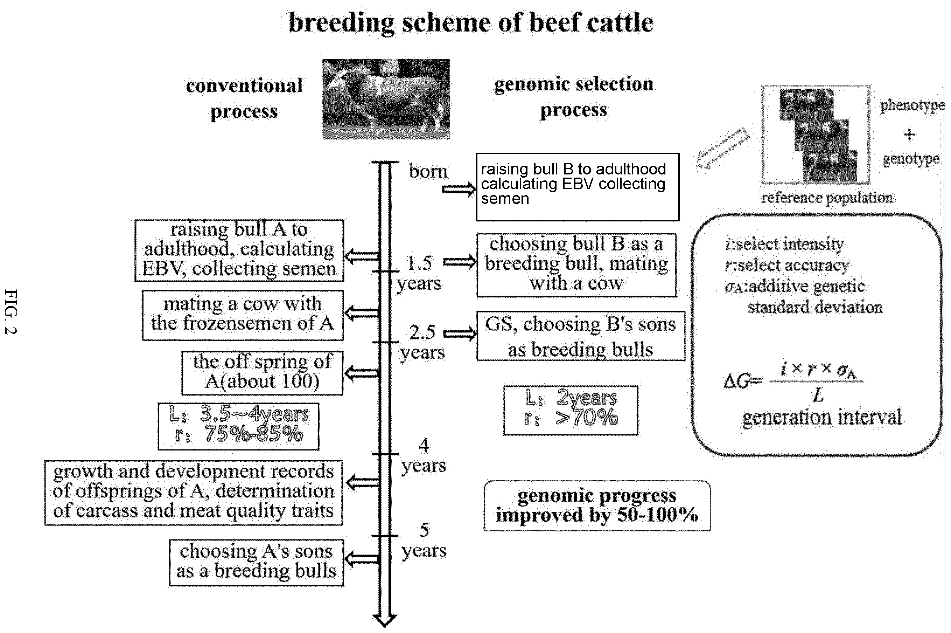

The breeding scheme of Huaxi breeding cattle is shown in FIG. 2. The average generation interval of Huaxi breeding cattle by using conventional breeding techniques is 3.5-4 years. If more than 100 descendants information are used, the reliability of breeding value estimation can reach 0.75, and it takes at least 5 years to cultivate an excellent Huaxi breeding cattle. However, an excellent Huaxi breeding bull can be cultivated in 2 years by using genomic selection technology, which greatly shortens the generation interval. Since blood can be collected from the candidate breeding cattle after birth to obtain the genotype, it is possible to use the genomic selection method of Huaxi cattle for early selection, which greatly saves the feeding cost and improves the selection efficiency and accuracy.

**EXAMPLE 2**

S1. establishment of reference population and determination of phenotypic traits: a team started to build the first Huaxi cattle resource population in China in Wulagai Management District of Xilingol League, Inner Mongolia since 2008. With the expanding of the population each year, by 2017, the number of basic cows of Huaxi cattle had exceeded 4,000. In July or August every year, the body size and weight data of calves and basic cows were measured in Wulagai management district, and the corresponding reproduction recording data were collected. In order to facilitate the measurement and collection of data on growth and development traits, slaughter traits, carcass traits and meat quality traits of the resource population in subsequent periods, Huaxi cattle aged from 5 to 9 months in Wulagai district were transported to a fattening and slaughtering integrated company in October every year, where each one of the Huaxi cattle should be measured for body weight and size data upon entry to the company. During concentrated fattening, weight and body size data are measured every three months. All the individuals in Huaxi cattle in this resource population were fattened intensively according to a unified feeding management method, and the feedstuff were all the same. When Huaxi cattle were fattened for 6 months, 20ml of venous blood was collected and stored, and 2ml of blood was used for extracting DNA (Deoxyribo Nucleic Acid). All individuals were treated with Illumina BovineHD(770K) high-density SNP (Single Nucleotide Polymorphism) chip to obtain the SNP data, and 44 representative Huaxi cattle were selected for whole genome resequencing. On the day before cattle slaughter, each one of the Huaxi cattle were fasting for 24 hours, and the live weight and body size before slaughter were measured. When Huaxi cattle of resource population were fattened intensively for 10-12 months, namely August to October of the following year, they were slaughtered in batches, and the slaughter and carcass data were obtained. Slaughter data includes all data of slaughtered cattle during slaughtering process, such as head weight, tongue weight, hoof weight, cowhide weight, kidney oil fat, red viscera (heart, liver, spleen, kidney) and white viscera (stomach) data, testicles and other slaughter data. After slaughter, all cattle carcasses were segmented after acid excretion for 48 hours, the segmentation data of the carcasses included the weight of beef clod, such as belly meat, neck meat, shin, money tendon, upper brain, eye meat, ectoloph, tenderloin, shank, topside, silverside. At the same time, in the process of carcass segmentation, meat samples (12-13 rib eye muscles, each about 1Kg) were collected for phenotypic data measurement of meat quality traits, which included shear force, water holding capacity(WHC), pH, marbling score, area of carcass eye muscle, intramuscular fat content, fatty acid traits of meat samples and the like. As a comprehensive index, weaning weight not only reflected the growth of calves before weaning, but also played a decisive role in the growth trend of calves after weaning. Calving difficulty reflected the reproductive ability of the female offspring of the cattle. Average daily gain during fattening was an important index to evaluate the growth and development of Huaxi cattle, in a sense, the average daily gain during fattening directly reflected the utilization of feedstuff by cattle. Carcass weight and dressing percentage were important indexes that directly reflected the relative carcass quality and production performance of beef cattle. Therefore, it was of great economic significance to select these five traits as important breeding target traits for selection and assortative mating to improve the production performance of beef cattle.

S2. genotyping and processing of chip data: 1047 indivuduals of Huaxi cattle were all genotyped with Illumina BovineHD (777962 SNPs) beadchip, and the SNP (42669 SNPs) on sex chromosome and plasmid were eliminated. The distribution of SNPs on each autosome in 1407 individuals was shown in Table 2, with 735293 SNPs in total.

Table 2. the number and ratio of SNPs on each autosome before quality control.

| chromosome | 1 | 2 | 3 | 4 | 5 | 6 | 7 | 8 | 9 | 10 |
|---|---|---|---|---|---|---|---|---|---|---|
| number of SNPs | 46495 | 40056 | 35579 | 34980 | 34842 | 35519 | 33168 | 33529 | 31060 | 30449 |
| ratio | 6.32% | 5.45% | 4.84% | 4.76% | 4.74% | 4.83% | 4.51% | 4.56% | 4.22% | 4.14% |

(continued)

| chromosome | 11 | 12 | 13 | 14 | 15 | 16 | 17 | 18 | 19 | 20 |
|---|---|---|---|---|---|---|---|---|---|---|
| number of SNPs | 32015 | 26127 | 23594 | 24780 | 24755 | 24178 | 22266 | 19386 | 18908 | 21490 |
| ratio | 4.35% | 3.55% | 3.21% | 3.37% | 3.37% | 3.29% | 3.03% | 2.64% | 2.57% | 2.92% |
| chromosome | 21 | 22 | 23 | 24 | 25 | 26 | 27 | 28 | 29 | |
| number of SNPs | 21175 | 18034 | 15215 | 18620 | 12931 | 15242 | 13152 | 13038 | 14710 | |
| ratio | 2.88% | 2.45% | 2.07% | 2.53% | 1.76% | 2.07% | 1.79% | 1.77% | 2.00% | |

Before data analysis, it was necessary to control the quality of genotype data, and eliminate the unqualified individuals and SNPs by using PLINK V1.9. The quality control standard and code in the application was: plink --cow --file filename --geno 0.1 --maf 0.05 --hwe 0.000001 --mind 0.1 --recode12 --out filename. After quality control, there were 1233 Huaxi cattle and 597031 SNPs remaining. After quality control, the chip data of Huaxi cattle population was imputed to the resequencing data of 44 Huaxi cattle, and the missing SNPs after quality control were filled with Beagle software. The running command of Beagle software was: java -Xmx1000m -jar beagle.jar unphased =file.bgl out = output niterations = 100, then the Beagle software was used to impute the SNPs by each chromosome into resequencing data (16165263 SNPs), running command was: java -d64 -Xss100m -Xmx100g -jar beagle.08Jun17.d8b.jar gt=chr1_vcf.gz ref=chr1.gt.vcf.gz ne=74 map=chr1_qc.map out=impute_chr1.gt nthreads=64. After imputation, the number and ratio of each autosome were shown in Table 3. The candidate QTLs were identified by using the imputed resequencing data for weaning weight, average daily gain in fattening, carcass weight, dressing percentage and calving difficulty, and then the identified QTLs were combined with beadchip data after quality control to form standard data for genomic selection of Huaxi cattle.

Table 3. The number and ratio of SNPs on each autosome of resequencing data

| chromosome | 1 | 2 | 3 | 4 | 5 | 6 | 7 | 8 | 9 | 10 |
|---|---|---|---|---|---|---|---|---|---|---|
| number of SNPs | 1040161 | 817823 | 753530 | 805070 | 760028 | 729866 | 696822 | 695580 | 618942 | 629850 |
| ratio | 6.43% | 5.06% | 4.66% | 4.98% | 4.70% | 4.52% | 4.31% | 4.30% | 3.83% | 3.90% |
| chromosome | 11 | 12 | 13 | 14 | 15 | 16 | 17 | 18 | 19 | 20 |
| number of SNPs | 687725 | 589685 | 568756 | 549805 | 594180 | 499293 | 469807 | 420515 | 408670 | 458459 |
| ratio | 4.25% | 3.65% | 3.52% | 3.40% | 3.68% | 3.09% | 2.91% | 2.60% | 2.53% | 2.84% |
| chromosome | 21 | 22 | 23 | 24 | 25 | 26 | 27 | 28 | 29 | |
| number of SNPs | 442247 | 382461 | 401819 | 450136 | 288791 | 364731 | 294293 | 346013 | 400205 | |
| ratio | 2.74% | 2.37% | 2.49% | 2.78% | 1.79% | 2.26% | 1.82% | 2.14% | 2.48% | |

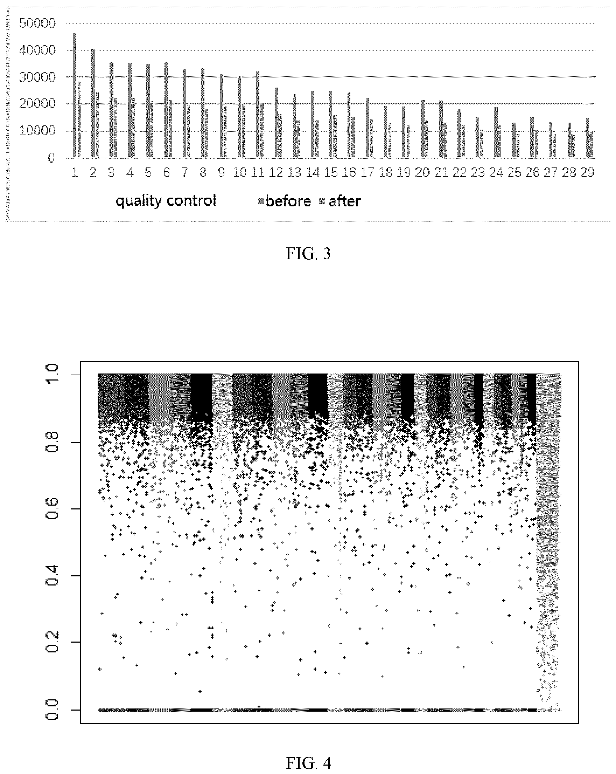

FIG. 3 shows the distribution of SNPs on each chromosome before and after quality control with beadchip in 1407 Huaxi cattle. It can be seen from FIG. 3 that unqualified SNPs on each chromosome are of the similar proportion.

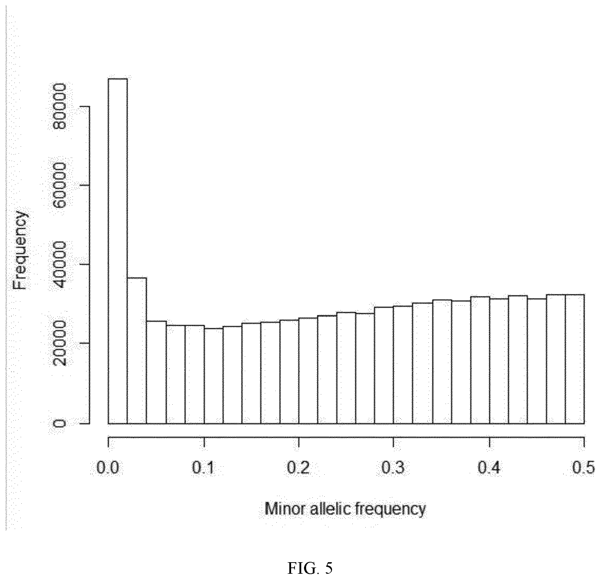

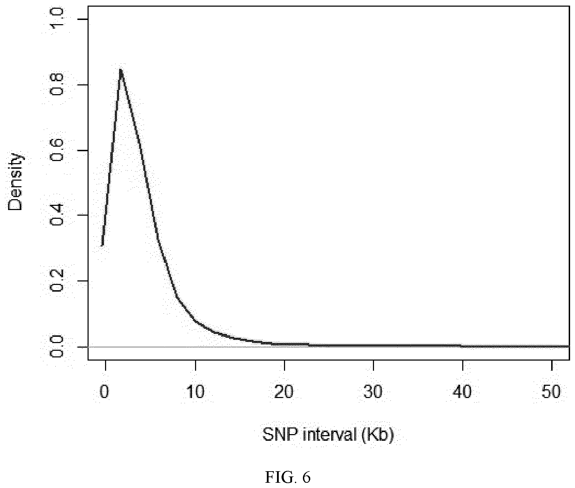

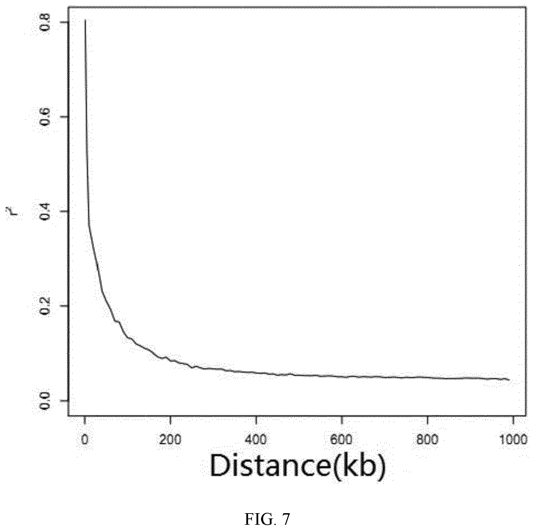

FIG. 4 shows the distribution map of the detected SNPs on each autosome for beadchip in Huaxi cattle population, indicating that most SNPs on autosomes are detected, and only a few are not detected. FIG. 5 shows the distribution map of the minor allele frequency (MAF) of all SNP markers (774,660 SNPs) for beadchip in Huaxi cattle population, it can be seen that the rare sites of the minor allele frequency (< 0.05) account for a certain proportion, after removing these sites, the MAF of the remaining markers are evenly distributed. FIG. 6 is a probability density map among SNPs markers of Illumina BovineHD chip, it can be seen from FIG. 6 that the majority of the distance between SNP markers is less than 10kb, and SNPs with a distance of 4kb account for the largest portion. FIG. 7 is the linkage disequilibrium map of Huaxi cattle population, it can be seen from FIG. 7 that the resource population of Huaxi cattle applied with beadchip has an average distance less than 10kb, which leads the population to a strong linkage disequilibrium state.

The method can make up for the blank of genomic selection of beef cattle in China, solve the problem of lack of genomic selection method in beef cattle production process, provide technical means for selecting cattle for breeding beef cattle with high-efficiency and high-quality in China, accelerate the process of beef cattle breeding in China, and facilitate the rapid development of beef cattle industry in China. The results show that the new breed of Huaxi cattle bred by the genomic selection method has a 50% growth compared with the conventional breeding technology. With regard to the largest reference population (2500 in total) of Huaxi cattle, the accuracy of genomic breeding value estimated by this self-developed new method (BayesBT) increase by 10-30%. This method can quickly estimate the genomic breeding value of Huaxi cattle in China, and has great application value and popularization prospect for beef cattle breeding in China.

The above described are only preferred embodiments of the present application, It should be understood by those skilled in the art that, without departing from the principle of the present application, any variations and modifications fall into the scope of the present application. Any appended drawing reference signs shall not be construed as limiting the claim.

In addition, it should be understood that although this specification is described according to embodiments, each embodiment does not contain only one independent technical scheme. The description of this specification is only for the sake of clarity, and those skilled in the art should take the specification as a whole. The technical schemes in each embodiment can also be combined appropriately to form other embodiments that can be understood by those skilled in the art. Nonetheless, the method is defined in the appended claims.

**Claims**

1. A genomic selection method of Huaxi cattle, comprising the following steps:

    S1. constructing a reference population and determining economic traits;
    S2. collecting and preserving blood for each individual in the reference population, extracting DNA and genotyping, quality control of the data after genotyping;
    S3. proceeding genotype imputation to the processed genotype data, obtaining a sequencing data, and then screening QTL loci;wherein the QTL loci comprise significant SNPs related to weaning weight, average daily gain during fattening, carcass weight, dressing percentage and calving difficulty;
    S4. integrating the identified QTLs with the high density beadchip genotyping data to obtain the standard data, calculating the effects of all SNP markers of each trait;
    S5. collecting blood and extracting DNA from a candidate population, genotyping using the biochip, proceeding imputation to the standard data after quality control, multiplying the genotype vector of the candidate population by the vector of SNP effects, wherein the effects of all SNP markers and QTLs were estimated by the reference population, thus obtaining the genomic estimated breeding value of the candidate population and calculating the genomic China beef index of the individual according to the genomic estimated breeding value; wherein the equation of individual comprehensive selection index(GCBI) used for the selection is as follows:

$$GCBI = 100 + (-5 \times \frac{Gebv_{CE}}{1.30} + 35 \times \frac{Gebv_{WWT}}{17.7} + 20 \times \frac{Gebv_{DG\_F}}{0.11} + 25 \times \frac{Gebv_{CW}}{16.4} + 15 \times \frac{Gebv_{DP}}{0.13}),$$

    wherein the $Gebv_{CE}$ is the genomic estimated breeding value of calving difficulty, $Gebv_{WWT}$ is the genomic estimated breeding value of weaning weight, $Gebv_{DG\_F}$ is the genomic estimated breeding value of average daily gain during fattening, $Gebv_{CW}$ is the genomic estimated breeding value of carcass weight, $Gebv_{DP}$ is the genomic estimated breeding value of dressing percentage.

2. The genomic selection method according to claim 1, wherein the quality control in S2 is eliminating the unqualified individuals and SNP, wherein the genotyping is carried out by using Illumina BovineHD beadchip.

3. The genomic selection method according to claim 1 or 2, wherein the economic traits in S1 comprise 6 indexes: growth and development, fattening, slaughter, carcass, meat quality and reproduction.

4. The genomic selection method according to claim 1, wherein using the Optimized Bayesian algorithm to calculate the effect value of all SNP markers of each trait, wherein in the optimized Bayesian algorithm the probability of SNPs with effect is (1-π), the probability of SNPs without effect is π, and the variance of effect obeys the inverse chi-square distribution, its model is as follows:

$$y = Xb + Wv + \sum_{i=1}^{n} Z_i g_i + e \quad,$$

wherein $y$ is a phenotypic observation vector and $b$ is a fixed effect vector; $W$ is the genotype association matrix of the identified trait QTL, and v is the locus effect value of the identified trait QTL, which is a fixed effect vector. $g_i$ is the $i^{th}$ marked effect value of the 770K beadchip, and the variance is $\sigma^2_{g_i}$; $n$ is the total number of marks; $e$ is the random residual vector with a variance of $\sigma^2_e I$, and $\sigma^2_e$ is the residual variance.


**Patentansprüche**

1. Verfahren zur genomischen Selektion von Huaxi-Rindern, umfassend die folgenden Schritte:

S1. Anlegen einer Referenzpopulation und Bestimmen wirtschaftlicher Merkmale;
S2. Entnehmen und Aufbewahren von Blut für jedes Individuum in der Referenzpopulation, Extrahieren von DNA und Genotypisieren, Qualitätskontrolle der Daten nach dem Genotypisieren;
S3. Ausführen von Genotypimputation an den prozessierten Genotypdaten, Erhalten von Sequenzierungsdaten, und dann Screenen von QTL-Loci; wobei die QTL-Loci signifikante SNPs in Bezug auf Absetzgewicht, durchschnittliche tägliche Zunahme während der Mast, Schlachtkörpergewicht, Ausschlachtung und Schwierigkeitsstufe der Kalbung umfassen;
S4. Integrieren der identifizierten QTLs mit den High-Density-Beadchip-Genotypisierungsdaten zwecks Erhalts der Standarddaten, Berechnen der Effekte aller SNP-Marker jedes Merkmals;
S5. Entnehmen von Blut und Extrahieren von DNA aus einer Kandidatenpopulation, Genotypisieren unter Verwendung des Biochips, Ausführen von Imputation an den Standarddaten nach Qualitätskontrolle, Multiplizieren des Genotypvektors der Kandidatenpopulation mit dem Vektor von SNP-Effekten, wobei
die Effekte aller SNP-Marker und QTLs durch die Referenzpopulation geschätzt wurden, wodurch der genomische geschätzte Zuchtwert der Kandidatenpopulation erhalten wird und der genomische China-Beef-Index des Individuums in Übereinstimmung mit dem genomischen geschätzten Zuchtwert berechnet wird; wobei die Gleichung des individuellen umfassenden Selektionsindex (GCBI), die für die Selektion genutzt wird, wie folgt lautet:

$$GCBI = 100 + (-5 \times \frac{Gebv_{CE}}{1{,}30} + 35 \times \frac{Gebv_{WWT}}{17{,}7} + 20 \times \frac{Gebv_{DG\_F}}{0{,}11} + 25 \times \frac{Gebv_{CW}}{16{,}4} + 15 \times \frac{Gebv_{DP}}{0{,}13}) \quad,$$

wobei der $Gebv_{CE}$ der genomisch geschätzte Zuchtwert für die Schwierigkeitsstufe der Kalbung ist, $Gebv_{WWT}$ der genomisch geschätzte Zuchtwert für Absetzgewicht ist, $Gebv_{DG\_F}$ der genomisch geschätzte Zuchtwert für durchschnittliche tägliche Zunahme während der Mast ist, $Gebv_{CW}$ der genomisch geschätzte Zuchtwert für Schlachtkörpergewicht ist, $Gebv_{DP}$ der genomisch geschätzte Zuchtwert für Ausschlachtung ist.

2. Verfahren zur genomischen Selektion nach Anspruch 1, wobei die Qualitätskontrolle in S2 die unqualifizierten Individuen und SNP eliminiert, wobei das Genotypisieren durch Verwendung von Illumina BovineHD Beadchip ausgeführt wird.

3. Verfahren zur genomischen Selektion nach Anspruch 1 oder 2, wobei die wirtschaftlichen Merkmale in S1 6 Indexe umfassen: Wachstum und Entwicklung, Mast, Schlachtung, Schlachtkörper, Fleischqualität und Reproduktion.

4. Verfahren zur genomischen Selektion nach Anspruch 1, wobei die Verwendung des optimierten Bayes-Algorithmus zur Berechnung des Effektwerts aller SNP-Marker jedes Merkmals, wobei im optimierten Bayes-Algorithmus die Wahrscheinlichkeit von SNPs mit Effekt (1-$\pi$) ist, die Wahrscheinlichkeit von SNPs ohne Effekt $\pi$ ist, und die Effektvarianz der Inverse-Chi-Quadrat-Verteilung folgt, sein Modell wie folgt lautet:

$$y = Xb + Wv + \sum_{i=1}^{n} Z_i g_i + e \quad,$$

wobei y ein Phänotypbeobachtungsvektor ist und b ein Fixer-Effekt-Vektor ist; W die Genotypassoziationsmatrix des identifizierten Merkmal-QTL ist, und v der Locus-Effekt-Wert des identifizierten Merkmal-QTL ist, was ein Fixer-Effekt-Vektor ist; $g_i$ der $i^{ter}$-Markierter-Effekt-Wert des 770K Beadchips ist, und die Varianz $\sigma_{gi}^2$ ist; $n$ die Gesamtzahl von Markierungen ist; $e$ der Random-Residual-Vektor mit einer Varianz $\sigma_e^2 I$ ist, und $\sigma_e^2$ die Residualvarianz ist.

## Revendications

1. Procédé de sélection génomique de bovins Huaxi, comprenant les étapes suivantes :

   S1. élaboration d'une population de référence et détermination de traits économiques ;
   S2. prélèvement et conservation de sang pour chaque individu dans la population de référence, extraction d'ADN et génotypage, contrôle qualité des données après génotypage ;
   S3. exécution de l'imputation de génotype aux données de génotype traitées, obtention de données de séquençage et ensuite criblage de loci QTL ; dans lequel les loci QTL comprennent des SNP significatifs liés au poids au sevrage, au gain quotidien moyen pendant l'engraissement, au poids de carcasse, au rendement de carcasse et à la difficulté de vêlage ;
   S4. intégration des QTL identifiés avec les données de génotypage par puce à bille haute densité pour obtenir les données standards, calcul des effets de tous les marqueurs SNP de chaque trait ;
   S5. prélèvement de sang et extraction d'ADN parmi une population candidate, génotypage en utilisant la biopuce, exécution de l'imputation aux données standards après un contrôle qualité, multiplication du vecteur de génotype de la population candidate par le vecteur d'effets SNP, dans lequel :

   les effets de tous les marqueurs SNP et QTL ont été estimés par la population de référence, obtenant ainsi la valeur reproductive estimée génomique de la population candidate et calculant ainsi l'indice de boeuf de Chine génomique de l'individu selon la valeur reproductive estimée génomique ; dans lequel l'équation de l'indice de sélection complète d'individu (GCBI) utilisé pour la sélection est comme suit :

$$GCBI = 100 + \left(-5 \times \frac{Gebv_{CE}}{1,30} + 35 \times \frac{Gebv_{WWT}}{17,7} + 20 \times \frac{Gebv_{DG\_F}}{0,11} + 25 \times \frac{Gebv_{CW}}{16,4} + 15 \times \frac{Gebv_{DP}}{0,13}\right) \quad,$$

   dans laquelle Ge$bv_{CE}$ est la valeur reproductive estimée génomique de difficulté de vêlage, Ge$bv_{WWT}$ est la valeur reproductive estimée génomique de poids au sevrage, Ge$bv_{DG\_F}$ est la valeur reproductive estimée génomique de gain quotidien moyen pendant l'engraissement, $Gebv_{CW}$ est la valeur reproductive estimée génomique de poids de carcasse, $Gebv_{DP}$ est la valeur reproductive estimée génomique de rendement de carcasse.

2. Procédé de sélection génomique selon la revendication 1, dans lequel le contrôle qualité dans S2 élimine les individus et les SNPs inappropriés, dans lequel le génotypage est réalisé en utilisant la puce à bille Illumina BovineHD.

3. Procédé de sélection génomique selon la revendication 1 ou 2, dans lequel les traits économiques dans S1 comprennent 6 indices : croissance et développement, engraissement, abattage, carcasse, qualité de la viande et reproduction.

4. Procédé de sélection génomique selon la revendication 1, dans lequel l'utilisation de l'algorithme bayésien optimisé pour calculer la valeur d'effet de tous les marqueurs SNP de chaque trait, dans lequel, dans l'algorithme bayésien optimisé, la probabilité de SNP avec un effet est (1-π), la probabilité de SNP sans effet est π et la variance d'effet obéit à la distribution chi carré inverse, son modèle est comme suit :

$$y = Xb + Wv + \sum_{i=1}^{n} Z_i g_i + e$$
,

où y est un vecteur d'observation phénotypique et *b* est un vecteur d'effet fixe ; *W* est la matrice d'association de génotype du QTL de trait identifié et v est la valeur d'effet de locus du QTL de trait identifié, qui est un vecteur d'effet fixe ; $g_i$ est la i$^{ème}$ valeur d'effet marqué de la puce à bille 770K et la variance est $\sigma_{gi}^2$ ; *n* est le nombre total de marques ; *e* est le vecteur résiduel aléatoire avec une variance de $\sigma_e^2 I$ et $\sigma_e^2$ est la variance résiduelle.

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016100061 A